# EUROPEAN PATENT APPLICATION

(11) **EP 3 498 254 A1**
(43) Date of publication of application: **19.06.2019**
(21) Application number: 17207703.4
(22) Date of filing: 15.12.2017
(51) Int. Cl.: A61K 8/20, A61K 8/23, A61K 8/25, A61K 33/14, A61K 33/04, A61Q 11/00

(54) **LIQUID MOUTHWASH, KIT COMPRISING SAID LIQUID MOUTHWASH AND USES THEREOF**

(71) Applicant: Stouten, Moniek, 53925 Kall-Benenberg (DE)
(72) Inventor: STOUTEN, Moniek, 53925 KALL-BENENBERG (DE); STOUTEN, Willem Frederik, 53925 KALL-BENENBERG (DE)
(74) Representative: van Dam, Vincent

(57) **Abstract**

The present invention relates to a liquid mouthwash composition, a kit comprising said liquid mouthwash and uses thereof. The mouthwash performs excellent with regard to dental care, and prolonged use of the mouthwash according to the invention has an advantageous effect on dental protection, in particular with regard to prevention of caries and successful prevention and treatment of gingivitis and/or periodontitis, without adverse painful experiences.

## Description

The present invention relates to a liquid mouthwash, a kit comprising said liquid mouthwash and uses thereof.

In addition to brushing teeth by means of a toothbrush with toothpaste, oral and dental hygiene liquid compositions are used which have an additional cleaning and caring effect due to the direct contact of the composition with the oral cavity. These compositions, commonly referred to as mouthwashes, ensure that even the most inaccessible areas of the oral cavity, especially the teeth, come into contact with the liquid composition.

While mouthwashes usually serve to refresh the mouth and pharynx and improve bad breath, there is a high demand for mouthwashes having a caring and whitening effect to the teeth.

In addition, there is also an increased aversion amongst consumers to take or ingest products with chemical additives, such as bleaching agents which have a whitening effect on the consumer's teeth.

The present invention therefore aims to provide a mouthwash composition which provides caring and whitening effects to the teeth, which is based on naturally occurring compounds and which does not contain additives that are detrimental to health and the environment.

### Description of the invention

The invention therefore relates to a liquid mouthwash, composed of a salt composition dissolved in water, wherein the salt composition comprises sodium chloride in an amount of 90 weight % or less of the salt composition, wherein said salt composition further comprises silicon containing minerals, and wherein silicon is present in the salt composition in an element mass proportion between 0,035 to 0,35 % by weight of the salt composition and wherein the liquid mouthwash comprises a salt concentration of between 5 and 15 % by weight of the total weight of the liquid mouthwash.

The inventor has surprisingly observed that a liquid mouthwash which is prepared by dissolving a salt composition which comprises a rather low sodium chloride concentration and silicon in the above specified range has excellent caring and whitening effects to the teeth.

The main component of the salt composition of the mouthwash is sodium chloride, which works as a gentle abrasive, and helps to remove stains from the teeth. In addition, sodium chloride has a disinfecting effect. Normal kitchen salt compositions often contain high concentrations of sodium chloride between 95 and 97 weight percent. If a consumer rinses his mouth with a solution of such salt, this results in an unpleasant and painful experience, especially when the subject is suffering from gingivitis or perionditis. The inventor now surprisingly discovered that when the ratio of sodium chloride to other salt component is 90 % by weight or less of the salt composition before dissolving it in water, consumers using a mouthwash according to the invention do not have the abovementioned unpleasant and painful experiences.

In order to achieve optimal effects regarding abrasive and stain removing action it is preferred that the salt composition comprises sodium chloride in an amount between 75 and 90 weight % of the solid salt composition before dissolving it in water, such as between 79,8 and 89,7 weight %.

Another important component of the salt composition of the mouthwash are silicon containing minerals. The inventor has observed that when silicon is present in the mouthwash of the invention in an element mass proportion between 0,035 to 0,35 % by weight of the solid salt composition before dissolving it in water, tooth decay and receding gums are reduced. Suitably, amounts of silicon may include the element mass proportion ranges of between 0.050- 0.30 % such as 0.052, 0.12, 0.27 % by weight of the salt composition. Silicon containing minerals may suitably be present in the form of silicon dioxide.

The combination of a relatively low sodium chloride content and the presence of silicon containing minerals in the mouthwash of the invention provides the users of the mouthwash with the experience of a soft and smooth feeling, in combination with a good cleansing effect and whitening of the teeth, without the need for chemical bleaching additives which may be detrimental for health and environment. The inventor has observed that prolonged use of the mouthwash according to the invention leads to improvement of tooth enamel, and prevention of caries. Prolonged use of the mouthwash according to the invention also results in the successful prevention and treatment of gingivitis and perionditis, without adverse painful experiences.

The salt composition is dissolved in the liquid mouthwash of the invention in a concentration of between 5 and 15 % by weight of the total weight of the liquid mouthwash. This way the mouthwash is provided as a ready-to-use composition which can be directly used by the consumer. In these concentrations, optimal user experience is provided. The best experiences have been obtained with a mouthwash comprising the salt composition in water in a concentration of about 10 % by weight of the total weight of the liquid mouthwash.

Apart from sodium chloride and silicon containing minerals, the mouthwash composition of the invention may suitably contain other elements, selected from the group of sulphur, magnesium, potassium, calcium, iron, aluminium and combinations thereof. Preferably the mouthwash contains all of these elements.

It is also preferred that the mouthwash of the invention contains trace elements. Trace elements may suitably be selected from the group of zinc, copper, erbium, tin, manganese, cerium, rubidium, gallium, titanium, praseodymium, bromine, strontium and combinations thereof. Preferably the mouthwash contains all of these elements in trace amounts. Trace elements are essential for all kinds of body processes and also improve dental health and can be present in the salt composition in their commonly used forms for human consumption.

In a preferred embodiment the salt composition used to prepare the mouthwash of the invention comprises sulphur in an element mass proportion of between 0.75 and 2,25 weight % of the salt composition. This results in improved healing of gums and reduced bleeding in case of gingivitis or periodontitis. Suitable exemplary element mass proportions of sulphur include 0.8 and 1.2 weight %. Sulphur containing compounds may suitably be present in the form of sulphates, such as sodium sulphate.

In another preferred embodiment the salt composition used to prepare the mouthwash of the invention comprises magnesium in an element mass proportion of between 0.25 and 3.5 weight % of the salt composition. This helps to strengthen the enamel, resulting in reduced risk of gingivitis and perionditis. Suitable exemplary element mass proportions of magnesium include 0.3, 0.4, 0.7, 1.1 and 3.1 weight %. Magnesium containing minerals may suitably be present in the form of magnesium chloride and/or magnesium bromide.

In another preferred embodiment the salt composition used to prepare the mouthwash of the invention comprises potassium in an element mass proportion of between 0.05 and 0.75 weight % of the salt composition. This contributes to a reduction of tooth sensitivity. Suitable exemplary element mass proportions of potassium include 0.1, 0.2, 0.23, 0.25, 0.5 and 0.6 weight %. Potassium containing minerals may suitably be present in the form of potassium chloride.

In another preferred embodiment the salt composition used to prepare the mouthwash of the invention comprises calcium in an element mass proportion of between 0.1 and 0.5 weight % of the salt composition. This strengthens the teeth. Suitable exemplary element mass proportions of calcium include 0.1, 0.13, 0.25, and 0.4 weight %. Calcium containing minerals may suitably be present in the form of calcium chloride.

Optimal results with regard to the abovementioned dental care effects have been observed with a liquid mouthwash according to the invention, wherein the salt composition comprises chloride in an element mass proportion of between 50 and 65 weight % of the salt composition, sodium in an element mass proportion of between 30 and 38 weight % of the salt composition, sulphur in an element mass proportion of between 0.75 and 2,25 weight % of the salt composition, magnesium in an element mass proportion of between 0.25 and 3.5 weight % of the salt composition, potassium in an element mass proportion of between 0.05 and 0.75 weight % of the salt composition, calcium in an element mass proportion of between 0.1 and 0.5 weight % of the salt composition, silicon in an element mass proportion of between 0.04 and 0.3 weight % of the salt composition, and trace elements, such as the trace elements mentioned above. Elements may suitably be present in the forms and exemplary proportions indicated above.

Element mass proportions as referred to in this application may be determined using non-destructive x-ray emission analysis.

It is preferred that the mouthwash does not contain bleaching additives. It is highly preferred that the mouthwash of the invention contains, apart from the salt composition and water, no further additives at all. Not only is it desirable that additives which may be detrimental for health and environment, such as whitening agents, are not included in the mouthwash, it is also not necessary include them to obtain the desired effects due to the excellent dental caring and whitening properties of the components of the salt composition.

The mouthwash of the invention preferably is slightly alkaline with a preferred pH between 8 and 9, such as between 8.1 and 8.6, such as between 8.15 and 8.5. This neutralizes acidity which promotes tooth and gum health. Normally the pH of the mouthwash will be slightly alkaline as a result of the salt composition used, so further adjustment of the pH will usually not be necessary.

The mouthwash of the invention can be easily prepared by dissolving the salt composition specified herein in water in the desired weight percentage.

As mentioned above the mouthwash of the invention is a ready-to-use mouthwash which can be directly used by a consumer. Consumers can easily pour a proper dosage (suitably between 10 and 20 ml) into a small dosage cup. In this respect the invention also relates to a kit comprising a container containing the mouthwash of the invention and a dosage cup. The contents of the cup can be poured in the consumer's mouth, after which it can be swished through the teeth for preferably about 30 to 60 seconds. It is recommended to swish the composition in front of and behind the teeth, through the molars as well as the front teeth and under the tongue and across the roof of the mouth. Although prior art mouthwashes in general should not be swallowed, it is not a problem at all if the consumer swallows the mouthwash of the invention, because as mentioned above, it does not contain harmful components. Nevertheless, the mouthwash of the invention may be suitably spit out into the sink after swishing.

As mentioned above, the mouthwash of the invention performs excellent with regard to dental care, and prolonged use of the mouthwash of the invention has an advantageous effect on dental protection. The liquid mouthwash according to the invention can therefore be used in dental protection. The inventor has observed in this respect that prolonged use of the mouthwash according to the invention contributes to successful prevention and treatment of caries and successful prevention and treatment of gingivitis and/or periodontitis, without adverse painful experiences.

The liquid mouthwash according to the invention can therefore in particular be used in a method of preventing or treating caries.

The liquid mouthwash according to the invention can also be used in a method of preventing or treating gingivitis and/ or periodontitis.

The inventor has also observed that prolonged use of the mouthwash according to the invention leads to whitening effects to the teeth. The invention therefore also relates to the use of the liquid mouthwash according to the invention in a cosmetic method for whitening teeth. In this method the composition is used in accordance with the recommendations for use as described above, i.e. by swishing the mouthwash through the teeth for an effective period of time, preferably between 30 and 60 seconds.

### Examples

In the following paragraphs it is illustrated how exemplary suitable embodiments of the mouthwash of the invention can be composed. These embodiments are meant to illustrate but not limit the invention.

Particularly suitable mouthwashes are composed of dissolved salt compositions having sodium chloride and silicon weight percentages as listed in Table 1. Weight percentages are based on the total weight of the salt composition before preparing the mouthwash.

**Table 1: suitable sodium chloride and silicon weight percentages.**

| Sodium chloride (weight percentage) | Element mass proportion of silicon (weight percentage) |
|---|---|
| 79,8 | 0,27 |
| 82,8 | 0,12 |
| 83,9 | 0,052 |
| 87,0 | 0,035 |
| 89,7 | 0,35 |

In a particularly suitable embodiment of the invention the liquid mouthwash composition is manufactured by dissolving a salt composition comprising the components in accordance with table 2 below. Weight percentages are based on the total weight of the salt composition before preparing the mouthwash.

**Table 2: components of a suitable salt composition for preparing a mouthwash according to the invention**

| Element | Element mass proportion - weight percentage (%) |
|---|---|
| Chloride | 50 - 65 |
| Sodium | 30 - 38 |
| Sulphur | 0.75 - 2.25 |
| Magnesium | 0.25 - 3.5 |
| Potassium | 0.05 - 0.75 |
| Calcium | 0.1 - 0.5 |
| Silicon | 0.04 - 0.3 |

In further particularly suitable embodiments of the invention the liquid mouthwash composition is manufactured by dissolving a salt composition comprising the components in accordance with tables 3 and 4 below. Weight percentages are based on the total weight of the salt composition before preparing the mouthwash.

**Table 3: components of a suitable salt composition for preparing a mouthwash according to the invention**

| Element | Element mass proportion - weight percentage (%) | Element | Element mass proportion - weight percentage (%) |
|---|---|---|---|
| Chloride | 50.90 | Zinc | 0.00275 |
| Sodium | 33.00 | Copper | 0,00195 |
| Sulphur | 0.820 | Erbium | 0,00195 |
| Magnesium | 0.441 | Tin | 0,00192 |
| Potassium | 0.227 | Manganese | 0.0018 |
| Calcium | 0.128 | Cerium | 0.00172 |
| Silicon | 0.052 | Fluoride | 0.00109 |
| Carbon | 0.049 | Rubidium | 0.00084 |
| Iron | 0.012 | Gallium | 0.00083 |
| Aluminum | 0.0095 | Borium | 0,00082 |
| Praseodymium | 0.0029 | Titanium | 0.00079 |
| Strontium | 0.00275 | Bromine | 0.00071 |

**Table 4: components of a suitable salt composition for preparing a mouthwash according to the invention**

| Element | Element mass proportion - weight percentage (%) | Element | Element mass proportion - weight percentage (%) |
|---|---|---|---|
| Chloride | 62,9 | Zinc | 0.0001 |
| Sodium | 31,4 | Copper | 0,0001 |
| Sulphur | 1,17 | Erbium | 0,0007 |
| Magnesium | 3,12 | Tin | 0,0059 |
| Potassium | 0,64 | Manganese | 0.0003 |
| Calcium | 0,41 | Cerium | 0.0023 |
| Silicon | 0,27 | Rubidium | 0.0007 |
| Iron | 0.0284 | Gallium | 0.0001 |
| Aluminum | 0.05 | Titanium | 0.0015 |
| Praseodymium | 0.0017 | Bromine | 0.04 |
| Strontium | 0.005 | | |

Element mass proportions in the above examples are determined using non-destructive x-ray emission analysis.

Mouthwashes composed of salt compositions according to the specifications in above tables 1, 2, 3 and 4 dissolved in water in a concentration 10% by weight of the mouthwash perform excellent with regard to dental care, and prolonged use of these mouthwashes has an advantageous effect on dental protection, in particular with regard to successful prevention and treatment of caries and successful prevention and treatment of gingivitis and/or periodontitis, without adverse painful experiences.

## Claims

1. A liquid mouthwash, composed of a salt composition dissolved in water,
wherein the salt composition comprises sodium chloride in an amount of 90 weight % or less of the salt composition,
wherein said salt composition further comprises silicon containing minerals, and wherein silicon is present in the salt composition in an element mass proportion between 0,035 to 0,35 % by weight of the salt composition
and wherein the liquid mouthwash comprises a salt concentration of between 5 and 15 % by weight of the total weight of the liquid mouthwash.

2. The liquid mouthwash according to claim 1, wherein the salt composition comprises sodium chloride in an element mass proportion between 75 and 90 weight % of the salt composition.

3. The liquid mouthwash according to claim 1 or 2, wherein the salt composition comprises sulphur in an element mass proportion of between 0.75 and 2.25 weight % of the salt composition.

4. The liquid mouthwash according to any of the claims 1 to 3, wherein the salt composition comprises magnesium in an element mass proportion of between 0.25 and 3.5 weight % of the salt composition.

5. The liquid mouthwash according to any of the claims 1 to 4, wherein the salt composition comprises potassium in an element mass proportion of between 0.05 and 0.75 weight % of the salt composition.

6. The liquid mouthwash according to any of the claims 1 to 5, wherein the salt composition comprises calcium in an element mass proportion of between 0.1 and 0.5 weight % of the salt composition.

7. The liquid mouthwash according to any of the claims 1 to 6, wherein the salt composition comprises chloride in an element mass proportion of between 50 and 65 weight % of the salt composition, sodium in an element mass proportion of between 30 and 38 weight % of the salt composition, sulphur in an element mass proportion of between 0.75 and 2.25 weight % of the salt composition, magnesium in an element mass proportion of between 0.25 and 3,5 weight % of the salt composition, potassium in an element mass proportion of between 0.05 and 0.75 weight % of the salt composition, calcium in an element mass proportion of between 0.1 and 0.5 weight % of the salt composition, silicon in an element mass proportion of between 0.04 and 0.3 weight % of the salt composition, and trace elements.

8. The liquid mouthwash according to any of the claims 1 to 7, wherein said salt composition is dissolved in water in a concentration of about 10 % by weight of the total weight of the liquid mouthwash.

9. The liquid mouthwash according to any of the claims 1 to 8 wherein the mouthwash does not contain bleaching additives.

10. The liquid mouthwash according to any of the claims 1 to 9, which is alkaline, and preferably with a pH between 8 and 9.

11. The liquid mouthwash according to any of the claims 1 to 10, for use in a method of preventing or treating caries.

12. The liquid mouthwash according to any of the claims 1 to 10, for use in a method of preventing or treating gingivitis.

13. The liquid mouthwash according to any of the claims 1 to 10, for use in a method of preventing or treating periodontitis.

14. Use of the liquid mouthwash according to any of the claims 1 to 10, for whitening teeth.

15. A kit, comprising at least a container containing the mouthwash according to any of the claims 1 to 10 and a dosage cup.
